Europäisches Patentamt

European Patent Office

Office européen des brevets

(11) Publication number: **0 125 690**
**B2**

# (12) NEW EUROPEAN PATENT SPECIFICATION

(45) Date of publication of the new patent specification: **14.11.90**

(21) Application number: **84105516.3**

(22) Date of filing: **15.05.84**

(51) Int. Cl.[5]: **C 07 C 51/48,** C 07 C 57/13, C 07 C 57/02

(54) Fractionation of polymerized fatty acids.

(30) Priority: **16.05.83 US 495096**

(43) Date of publication of application:
**21.11.84 Bulletin 84/47**

(45) Publication of the grant of the patent:
**29.10.86 Bulletin 86/44**

(45) Mention of the opposition decision:
**14.11.90 Bulletin 90/46**

(84) Designated Contracting States:
**DE FR GB NL**

(56) References cited:
CA-A- 366 525   US-A-3 969 196
DE-A-2 843 920   US-A-4 308 200
DE-B-1 069 629   US-A-4 568 495
GB-A-1 386 396

(73) Proprietor: **Union Camp Corporation**
**1600 Valley Road**
**Wayne New Jersey 07470 - 2066 (US)**

(72) Inventor: **Frihart, Charles Richard**
**13 Winnipeg Lane**
**Lawrenceville, NJ 08648 (US)**

(74) Representative: **von Füner, Alexander, Dr. et al**
**Patentanwälte v. Füner, Ebbinghaus, Finck**
**Mariahilfplatz 2 & 3 Postfach 95 01 60**
**D-8000 München 95 (DE)**

(56) References cited:
McHugh, M. A. and Krukonis, Val J.,
"Supercritical Fluid Extraction, Principles and
Practices", Butterworths, Boston, 1986
National Research Council, "International
Critical Tables of Numerical Data, Physics,
Chemistry and Technology", McGraw Hill, New
York, Volume III,1928
"Bailey's Industrial Oil and Fat Products", 1964,
Chapter 12, pp. 491-514
Angew. Chemie, Int. Ed. Engl. 17 (1978), pp.
702-709

Courier Press, Leamington Spa, England.

**Description**

BACKGROUND OF THE INVENTION
Field of the Invention

The invention relates to methods for the fractionation of polymeric mixtures and more particularly relates to methods of separating the various polymer components of polymerized fatty acids.

Brief Description of the Prior Art

Polymeric fatty acids are prepared by the polymerization of fatty acids, using techniques well known to those skilled in the art; see for example the polymerization described in U.S. Patent 3,157,681. These polymeric fatty acids are sometimes referred to in the art as "dimer fatty acids" because the predominant constituent is the dimer of the fatty acid. However, the polymerized fatty acids are complex mixtures, containing unreacted monomeric compounds, trimer forms and higher polymeric forms of the fatty acids.

Commercially available polymeric fatty acids prepared from tall oil fatty acids are representative of polymeric fatty acids and may have a composition as follows:

|  | % BY WEIGHT |
| --- | --- |
| $C_{18}$ monobasic acids (monomer) | 0—5 |
| $C_{36}$ dibasic acids (dimer) | 60—95 |
| $C_{54}$ and higher polybasic acids (trimer) | 1—35 |

The relative ratios of monomer, dimer and trimer in unfractionated polymeric fatty acids are dependent on the nature of the starting material and the conditions of polymerization.

Heretofore, fractionation of the polymeric fatty acids to separate the monomer and dimer-trimer components has been done by conventional distillation and molecular distillation, and the separation of dimer and trimer components only by molecular distillation. These techniques are of course costly procedures and are adversely impacted upon by any ash in the dimerization product.

The method of the present invention provides a relatively inexpensive means to fractionate polymeric fatty acids.

The employment of solvent gases as liquids in their near critical state and as fluids in the supercritical state as extracting solvents has been previously described; see for example Francis, Physical Chem, 58,1099 (1954) and Ind. Eng. Chem. 47, 230 (1955). Near critical and supercritical fluids, including hydrocarbon gases have been suggested as solvents for a wide range of materials; see for example U.S. Patent 3,969,196. Despite the fact that the solvation properties of gases in their near critical and supercritical states and especially of hydrocarbon gas, has been known, the application of this knowledge has not been applied to the fractionation of polymeric fatty acids.

In CA—A—366 525 there is described a process for separating a high molecular mixture containing high molecular non-hydrocarbon compounds into portions having different properties, comprising the steps of dissolving said mixture in a quantity of a liquid low molecular treating agent to produce a liquid phase, said treating agent being capable of dissolving a component of the mixture when the treating agent is in its paracritical state and precipitating another component, heating said liquid phase to a temperature at which the treating agent is in its para-critical state under a pressure not substantially less than the vapor pressure of the liquid phase, thereby causing the precipitation of a portion of the mixture and the formation of a separate heavier phase, and separating the heavier phase from the lighter liquid phase.

In US—PS 3,969,196 there is described a process of separating a mixture which is in liquid state or solid state or liquid and solid state composed of a plurality of compounds at least one of which contains an organic group which comprises:

a. contacting said mixture with a gas phase preferentially taking up said compound containing an organic group at the contacting conditions, said gas phase during said contacting being maintained under supercritical conditions of temperature and pressure such that the gas takes up at least a portion of said compound containing an organic group, the temperature being in a range in which the quantity of said compound containing an organic group taken up by said gas phase varies inversely with said temperature, and effecting said contacting in a manner so that this occurs, and so that there is a substantial gas component that is identifiable as a gaseous component, the critical temperature of said gas phase being in the range of 0°C—200°C, the temperature of said gas phase during said contacting being within about 100°C above the critical temperature,

b. separating the gas phase in the form of said identifiable gaseous component loaded with said portion of the compound containing an organic group taken up during said contacting from any of the mixture not taken up by the gas phase while still maintaining supercritical conditions as aforesaid,

c. thereafter separating at least part of the compound containing an organic group taken up, from the gas phase.

Moroever, GB—PS 1 386 396 refers to a process for the separation of fatty acids which process comprises mixing the fatty acids with an organic solvent for the fatty acids and cooling the mixture by direct expansion at atmospheric pressure of liquefied refrigerating agent, which refrigerating agent is gaseous at atmospheric pressure to cause the acid of higher freezing point to crystallise while the other acid or acids remain in solution.

## SUMMARY OF THE INVENTION

The invention comprises a method of fatty acid monomer, dimer and trimer from polymerized fatty acid mixtures containing said monomer, dimer and trimer, characterized in that the mixture is contacted with carbon dioxide in form of a supercritical fluid sovlent at a temperature of ca. 60°C and under pressure of 225 to 455 bar leaving a first residue, which is contacted with a second supercritical fluid solvent namely ethylene at a temperature of ca. 70°C and under pressure of 140 to 350 bar leaving a second residue, which is contacted with the third supercritical fluid namely the supercritical propylene at a temperature of ca. 107°C under a pressure of 140 to 350 bar.

## BRIEF DESCRIPTION OF THE DRAWING

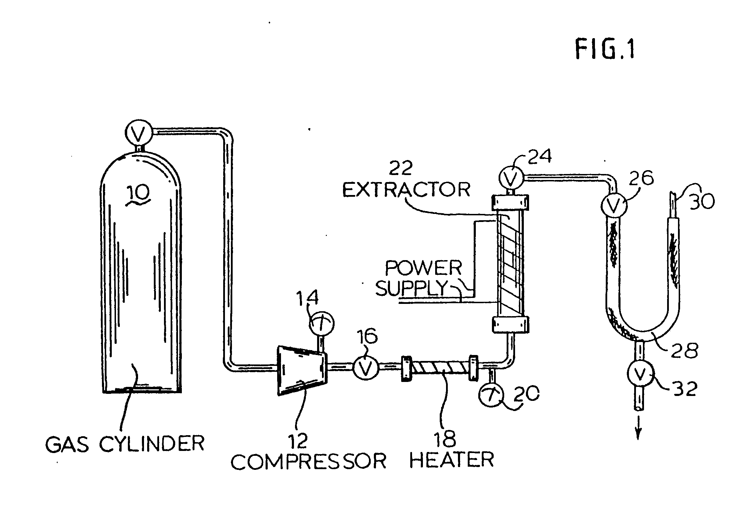

The accompanying drawing of Figure 1 is a schematic representation of apparatus employed in the method of the invention to fractionate polymeric fatty product with supercritical fluid solvents.

## DETAILED DESCRIPTION OF THE PREFERRED EMBODIMENTS OF THE INVENTION

The method of the invention is carried out by bringing the polymeric fatty acid mixture in contact with the respective supercritical fluid solvent so as to fractionate the monomer, dimer and trimer components based upon their solubility in the supercritical fluid solvents. A wide variety of gases which are solvents in the supercritical fuid state may be employed. Representative of such gases are the hydrocarbon gases such as methane, ethane, propane, butane, ethylene, propylene and the like. Preferably, hydrocarbon gases are employed as the supercritical solvent fluids to fractionate dimer and trimer. Carbon dioxide may also be used in the supercritical fluid state and is preferred to fractionate the monomer compounds. The conditions under which such gases become supercritical fluids are well known to those skilled in the art as is apparatus for their manufacture and use in the extraction of organic materials; see for example the teachings set forth in the U.S. Patents 3 969 196 and 4 308 200. The supercritical fluids are brought into contact with the polymeric fatty acids in a suitable vessel, under supercritical fluid conditions. Preferably, hydrocarbon gases are employed as the supercritical solvent fluids.

The Figure 1 of the accompanying drawing is a schematic representation of an apparatus, simple in nature, which may be employed in the method of the invention and because of its simplicity serves to exemplify the method of the invention. As shown in the accompanying drawing, a source of a suitable gas in the cylinder 10 may be fed by conduits into a compressor 12 wherein it is compressed to supercritical pressure conditions. The gas selected is one which in the supercritical state will preferentially dissolve one of the monomer, dimer or trimer components of the polymerized fatty acid over the remaining components. Preferably the pressure is within the range of from about 210 to 350 bar. A pressure gauge 14 monitors the compression and valve 16 provides a means for delivery of the compressed fluid to a heater 18 where the temperature of the compressed fluid is raised to a supercritical temperature. Preferably the temperature is within the range of from about 0° to about 200°C. Temperature gauge 20 monitors the heating of the compressed fluid which is then passed into an extractor vessel 22 which is provided with an electrical resistance heater means for maintaining the supercritical temperature. Previous to introduction of the supercritical gas into extractor vessel 22, the vessel 22 is charged with a quantity of polymeric fatty acid for extraction. When the supercritical fluid selected is one which will dissolve monomer compounds from the polymerized fatty acid mixture and it is introduced into the charged extractor vessel 22, extraction of monomer from the polymer mixture begins immediately. The monomer extract with the supercritical fluid is carried through expansion valves 24 and 26 to reduce the supercritical pressure of the gas. Upon dropping the pressure of the mixture below the supercritical pressure for the fluid, the extracted monomer is dropped from solution in the supercritical fluid, remaining in the separator tube 28 as the reduced pressure gaseous solvent is vented through end 30 of separator tube 28. Of course, the vented gas can be reused in a continuous process by recycling the gas back to the compressor 12 through outlet 30. The extracted liquid monomer product is then removed from the apparatus by opening valve 32. This process may be continued for a sufficient time to remove most of the monomer. Then the pressure or temperature of the supercritical extraction fluid may be increased or the fluid itself is changed so as to preferentially extract the dimer following the general procedure described above. The final changes in conditions of higher pressure or temperature or of a different supercritical fluid solvent then allows for the isolation of trimer, again following the same general procedure described above.

The following example describes the manner and process of making and using the invention and sets forth the best mode contemplated by the inventor for carrying out the invention but is not to be construed as limiting.

An experiment to fractionate crude polymerized fatty acid using several supercritical fluids was conducted employing the apparatus described above and shown in the accompanying drawing of Figure 1. The extractor vessel 22 was loaded with 5.93 grams of polymerized fatty acid shown by gas-phase chromatography to comprise 2.7 percent monomer, 80.7 percent dimer and 16.6 percent trimer (% peak height). The charged extractor vessel was heated to a temperature of 60°C and the compressed carbon dioxide at a pressure of 225 bar, heated to a temperature of 60°C, was allowed to enter the extractor vessel and contact the crude polymer mixture present. The compressed gas was passed through the extractor vessel and then allowed to expand at 1 atmosphere pressure immediately prior to entering the separating tube 28 described above. Visual observation of material being deposited in the separator was an indication of material solubility and extraction.

The above procedure was repeated a number of times, on the residue of the preceding charge but employing a variety of temperatures, pressures and supercritical fluids. The gases, temperatures, pressures and extracts obtained are reported in the TABLE below.

TABLE

ANALYSIS

| Gas Used | Temp. (°C) | Pressure (bar) | Amount Extracted (gm) | % Peak Height (GPC) Relative | | |
|---|---|---|---|---|---|---|
| | | | | Monomer | Dimer | Trimer |
| $CO_2$ | 60 | 225 | 0.09 | 29.2 | 70.8 | — |
| $CO_2$ | 60 | 260—385 | 0.35 | — | 96.6 | 3.4 |
| $CO_2$ | 70 | 455 | 0.19 | 13.3 | 80.7 | 6 |
| $C_2H_4$ | 70 | 140—350 | 0.98 | — | 93.7 | 6.3 |
| $C_2H_4$ | 70 | 385 | 2.4 | — | 90.2 | 9.8 |
| $C_2H_4$ | 77 | 385 | 1.56 | — | 80.4 | 19.6 |
| $C_3H_8$ | 107 | 140—350 | 0.5 | — | 24.2 | 75.8 |
| $C_3H_8$ | 107 | 350 | 0.1 | — | 22.8 | 77.2 |

**Claim**

Method of separating fatty acid monomer, dimer and trimer from polymerized fatty acid mixtures containing said monomer, dimer and trimer, characterized in that the mixture is contacted with carbon dioxide in form of a supercritical fluid solvent at a temperature of ca. 60°C and under pressure of 225 to 455 bar leaving a first residue, which is contacted with a second supercritical fluid solvent namely ethylene at a temperature of ca. 70°C and under pressure of 140 to 350 bar leaving a second residue, which is contacted with the third supercritical fluid namely the supercritical propylene at a temperature of ca. 107°C under a pressure of 140 to 350 bar.

**Patentanspruch**

Verfahren zur Abtrennung eines Fettsäuremonomers, -dimers und -trimers aus polymerisierten Fettsäuregemischen, enthaltend das Monomer, Dimer und Trimer, dadurch gekennzeichnet, daß das Gemisch mit Kohlendioxid in Form eines überkritischen flüssigen Lösungsmittel bei einer Temperatur von ca. 60°C und einem Druck von 225 bis 455 bar kontaktiert wird, wobei ein erster Rest zurückbleibt, der mit einem zweiten überkritischen flüssigen Lösungsmittel, nämlich Ethylen, bei einer Temperatur von ca. 70°C und einem Druck von 140 bis 350 bar kontaktiert wird, wobei ein zweiter Rest zurückbleibt, der mit der dritten überkritischen Flüssigkeit, nämlich dem überkritischen Propylen, bei einer Temperatur von ca. 107°C und einem Druck von 140 bis 350 bar kontaktiert wird.

**Revendication**

Un procédé de séparation d'acides gras monomère, dimère et trimère à partir de mélanges d'acides gras polymérisés contenant lesdits monomère, dimère et trimère caracterisé en ce que le mélange est contacté avec du dioxyde de carbone en forme d'un solvant fluide super-critique à une température d'environ 60°C et sous une pression de 225 à 455 bars, en laissant un premier résidu, qui est contacté avec un second solvant fluide super-critique, à savoir de l'éthylène, à une température d'environ 70°C et sous une pression de 140 à 350 bars, en laissant un second résidu, qui est contacté avec le troisième fluide super-critique, à savoir du propylène super-critique, à une température d'environ 107°C et sous une pression de 140 à 350 bars.

FIG.1

POWER SUPPLY

22 EXTRACTOR

10

GAS CYLINDER

12 COMPRESSOR

18 HEATER

EP 0 125 690 B2